## Europäisches Patentamt
## European Patent Office
## Office européen des brevets

(11) Publication number: **0 102 200**
**B1**

# (12) EUROPEAN PATENT SPECIFICATION

(45) Date of publication of patent specification: **04.02.87**

(51) Int. Cl.[4]: **A 61 K 6/02,** A 61 K 7/18

(21) Application number: **83304430.8**

(22) Date of filing: **01.08.83**

(54) **Neutral topical sodium fluoride gel.**

(30) Priority: **02.08.82 US 404263**

(43) Date of publication of application:
**07.03.84 Bulletin 84/10**

(45) Publication of the grant of the patent:
**04.02.87 Bulletin 87/06**

(84) Designated Contracting States:
**AT BE CH DE FR GB IT LI NL SE**

(56) References cited:
**EP-A-0 007 170**
**GB-A-1 450 881**
**GB-A-1 551 388**
**US-A-3 337 412**

**PATENTS ABSTRACTS OF JAPAN, vol. 4, no.**
**129 (C-24)611r, 10th September 1980, page 129**
**C 24**

The file contains technical information
submitted after the application was filed and
not included in this specification

(73) Proprietor: **JOHNSON & JOHNSON DENTAL**
**PRODUCTS COMPANY**
**20 Lake Drive CN 7060**
**East Windsor New Jersey 08520 (US)**

(72) Inventor: **Zahradnik, Robert Thomas**
**1-07 Hunters Glen Drive**
**Plainsboro New Jersey 08536 (US)**

(74) Representative: **Jones, Alan John et al**
**CARPMAELS & RANSFORD 43 Bloomsbury**
**Square**
**London, WC1A 2RA (GB)**

(56) References cited:
**CHEMICAL ABSTRACTS, vol. 92, no. 5, 4th**
**February 1980, no. 33718t, Columbus, Ohio, US**
**U. HEINTZE et al.: "Accumulation and**
**clearance of fluoride in human mixed saliva**
**after different topical fuoride treatments"**

**CHEMICAL ABSTRACTS, vol. 74, no. 26, 28th**
**June 1971, page 312, no. 146414n, Columbus,**
**Ohio, US**

Courier Press, Leamington Spa, England.

## Description

The invention relates to a neutral topical sodium fluoride gel composition useful for the control and prevention of dental caries when topically applied to erupted teeth.

Background of the invention

Neutral aqueous solutions of sodium fluoride were first used in the prevention of dental caries about 40 years ago. The initial clinical studies using neutral sodium fluoride were carried out by Knutson and Armstrong (Public Health Rep. *58*, 1701 (1943)) and by Bibby (J. Am. Dent. Assoc. *31*, 317 (1944)) In the early 1960's, acidulated phosphate fluoride ("APF") appeared to be a preferred material in that some laboratory tests by Brudevald et al. (Arch. Oral Biol. *8*, 167 (1963)) indicated that the APF System gave a higher fluoride uptake than neutral sodium fluoride. Therefore, the predominant fluoride material used by dentists, hygienists, and other dental technicians since then has been the APF material. In the latter 1960's, the APF material was introduced in the form of a gel. One such formulation is described by Elbreder in US—A—3,337,412. Another APF gel is described by Weitzman et al., in US—A—4,267,167.

Until this invention was made, as far as is known to the inventor the only pre-mixed fluoride gel intended for use by dental professionals that has been available commercially has been the APF gel.

There have been disclosures in the literature of certain neutral sodium fluoride gels that apparently were either mixed by the user or by the investigator. For instance, see JASPD January—February 1975, 17—21, 36—45 (especially pages 38 and 40) (anon) "Everything You Always Wanted To Know About Fluoride Therapy", and references cited therein, especially: Englander et al., J. Am. Dent. Ass. 75: 639, 1967; Hayward et al., "Ca-A Cancer Journal For Clinicians". 13—21, April-May 1969; Cole et al., J. Dent. Res. Special Issue—Program and Abstracts 52: 246, 1973; Englander et al., J. Am. Dent. Ass. 78: 783, 1969; Carter, "Midwest Dentist", 43:17, 1967; and Law, J. Am. Dent. Ass. 73:835, 1966.

The only gelling agents specifically disclosed in these articles for use in neutral sodium fluoride gels are cellulose derivatives, either hydroxypropyl methyl cellulose or carboxymethyl cellulose.

JP—A—55—83709 (mentioned in Patent Abstracts of Japan, *4*, 129 (C-24), 611, 1980) discloses a fluorine-containing preparation for prevention of tooth decay comprising a water-soluble polymer, such as gum arabic, methyl cellulose or polyvinyl pyrrolidone, and a fluorine compound. Other components such as softeners may be included if desired.

GB—A—1 450 881 discloses a fluoride ion-containing gel for use in preventive dentistry comprising an aqueous solution of a water-soluble source of fluoride ions gelled with xanthan gum. No other thickening agent is mentioned in the disclosure. A number of the Examples apparently show neutral compositions.

Despite these disclosures in the literature of certain neutral sodium fluoride gels, the dental profession had continued to use the APF gels for topical fluoride application. This is surprising, since, from the standpoint of clinical efficacy, it now is believed that there is no difference between neutral sodium fluoride and APF (For review, see Ripa, Intl. Dent. J. *31*, 105 (1981)) and yet there are some disadvantages to the use of the APF gel. For instance, APF has an acid etching potential for porcelain, polymer-glass composite materials, and for metal bridge-work and orthodontic brackets. This could lead to staining, weakening, and reduction of polish.

Up to now, most professional fluoride "gel" treatments have been carried out on pre-teenage children. On these patients, the acid etching potential of APF gel is not a significant problem because they have a low incidence of porcelain, composites and metal bridgework. However, the dental profession has come to recognize that fluoride treatment would be beneficial to many more patients than pre-teenagers. For instance fluoride treatment to control and/or prevent caries is useful for those undergoing orthodontic treatment, children as well as adults. Because of improved preventive and treatment measures, more adults are retaining their natural teeth; therefore, coronal and root dental caries is becoming a significant health problem in the adult population (U.S. National Center for Health Statistics data, 1971—1974.) Adult patients suffering from gum disease such as gingivitis and periodontal disease would benefit from fluoride treatment to retard the formation of caries in the portions of the roots of teeth that are becoming exposed because of receding gum lines. Also, people who are handicapped, people with impaired salivary flow caused by head and neck radiation, and hemophiliacs can benefit from fluoride treatment to control dental caries. However, when fluoride gel treatment is extended to such patients, the acid etching potential of APF gel can become a significant concern.

The present invention is based upon the discovery that a neutral sodium fluoride gel using a specified combination of thickeners or gelling agents is particularly well adapted for use in dental caries prevention and control.

The present invention provides a thickened sodium fluoride aqueous composition suitable for use in the topical prophylactic treatment of teeth, said composition comprising 75.2 to 96.1% w/w water, 1 to 5% w/w sodium fluoride, and thickener consisting of xanthan gum and a water soluble salt of an acrylic acid polymer, said composition having a pH of 6 to 8.

The neutral topical sodium fluoride gel of the invention is actually a thickened solution, and is not a true gel. However, because the word "gel" is widely employed in the dental profession to refer to such materials, it will be used herein, with the understanding that the invention actually relates to a thickened solution and not to a true crosslinked "gel".

The gel of the invention includes water, sodium fluoride, thickener, and preferably a buffer to maintain the pH of from 6 to 8. Optionally, the composition will also contain flavoring agents, sweeteners, preservatives, and coloring agents at levels necessary to achieve the desired effects. Thus, typical ranges of proportions of the ingredients are shown in the following Table 1:

TABLE 1
Basic formulation

| Ingredient | % (w/w) |
|---|---|
| Water | 75.2 —96.1 |
| Sodium Fluoride | 1.0 — 5.0 |
| Thickener | 1.0 —20.0 |
| Buffer | 0.5 — 1.5 |
| Flavor | 0.1 — 0.5 |
| Sweetener | 0.1 — 0.3 |
| Preservatives | 0.15— 0.35 |
| Coloring | 0.05— 0.15 |

The thickener that is employed in the invention comprises a mixture of xanthan gum and a water-soluble salt of an acrylic acid polymer, e.g. the sodium salt of polyacrylic acid.

The thickener is safe for human ingestion (FDA approved for internal use), does not bind fluoride ion or in any way significantly affect its bioavailability, and provides the desired flow characteristics to the products of this invention.

The thickener is added in a proportion sufficient to achieve a solution viscosity adequate to maintain a gel in an inverted mouthpiece tray. applicator for up to about four minutes (typical time for a professionally applied fluoride treatment), and yet be fluid enough to have acceptable handling characteristics for the dental operator (e.g. when dispensing into a gel tray applicator). The thickening agent, therefore, must impart a certain degree of thixotropy to the neutral sodium fluoride formulation such that at rest the product has a thick, gel-like consistency, but under a shearing force, resulting perhaps from simple agitation, the sodium fluoride gel thins or undergoes a reversible transition to a more fluid condition.

Routine experimentation will suffice to determine the exact proportion of thickener needed to impart the above characteristics. The Example below illustrates typical proportions for the thickener.

The preferred formulation for use in the invention is shown below in Table II:

TABLE II
Preferred formulation for the neutral sodium fluoride "gel" composition

| Ingredient | % (w/w) |
|---|---|
| Water | 87.7 |
| Sodium Fluoride | 2.0 |
| Carbopol 934P (Polyacrylic acid) | 1.2 |
| Xanthan Gum | 1.2 |
| Sodium Hydroxide, 10% [aqueous] | 6.5 |
| Disodium Phosphate, Anhydrous | 0.7 |
| Flavor | 0.3 |
| Sodium Saccharin | 0.1 |
| Sodium Benzoate | 0.2 |
| Methyl Paraben | 0.05 |
| Coloring Solution | 0.05 |
| | 100.00 |

pH=7.0
Viscosity=8.5 Pa.S (85 poise) at 60 Rpm and 25°C.

Carbopol 934P is polymerized acrylic acid. The aqueous sodium hydroxide is added to neutralize this material to form the sodium salt thereof.

The pH of the gel can be adjusted to the desired range by adding acids or bases such as phosphoric acid, hydrochloric acid, or sodium hydroxide, which form salts that are safe to ingest, or by the addition of ingestible buffering agents such as sodium phosphates. Disodium phosphate and monosodium phosphate are the preferred buffers. The buffer is preferred in order to maintain the pH within the desired neutral range, since absorption of carbon dioxide from air could reduce the pH of an unbuffered gel.

The thickening agent is employed in a proportion sufficient to impart a viscosity of from 4 to 20 Pa.S (40 to 200 Poise) to the solution tested by a Brookfield Viscometer at 60 rpm and 25°C, using Spindle No. 5. The degree of thixotropy of the gel is preferably such that the Brookfield viscosity measured at 1.5 rpm and 25°C, using Spindle No. 4, is within the range of from 50 to 400 Pa.S (500 to 4,000 poise).

The thickened neutral sodium fluoride aqueous solutions of the invention are applied to the teeth by the dentist, hygienist, or technician in the conventional manner. This involves either a tray technique or a "paint on" technique. The tray technique is preferred because of the convenience and time-saving aspects of the tray application. In this method, a gel application tray is filled approximately one-third full with the gel product. After the teeth are thoroughly dried, the filled tray is inserted into the patient's mouth, and is maintained there for four minutes. The tray is then removed and the patient expectorates any excess gel remaining in the mouth. For the "paint on" technique, the teeth are isolated with cotton rolls and dried thoroughly with air. A cotton applicator is then dipped into the gel and applied to the teeth, keeping the teeth covered with gel for four minutes. Once again, after the cotton rolls are removed, the patient expectorates excess gel.

**Claims**

1. A thickened sodium fluoride aqueous composition suitable for use in topical prophylactic treatment of teeth, said composition comprising 75.2 to 96.1% w/w water, 1 to 5% w/w sodium fluoride, and thickener consisting of xanthan gum and a water soluble salt of an acrylic acid polymer, said composition having a pH of 6 to 8.

2. The composition of claim 1, further including a buffering agent.

3. The composition of claim 2, wherein the buffering agent is disodium phosphate, monosodium phosphate or a mixture thereof.

4. The composition of any one of claims 1 to 3, further including flavoring agents, preservatives or colouring agents.

5. The composition of any one of claims 1 to 4, which has a viscosity of from 4 to 20 Pa.S (40 to 200 poise) tested by a Brookfield viscometer at 60 RPM and 25°C, and a viscosity of 50 to 400 Pa.S (500 to 4000 poise) tested by a Brookfield viscometer at 1.5 RPM and 25°C.

6. The composition of any one of claims 1 to 5, wherein the thickener is xanthan gum plus the sodium salt of polyacrylic acid.

7. A composition according to any one of claims 1 to 6 for use in controlling dental caries.

8. A method of preparing a composition according to any one of claims 1 to 7 comprising mixing said ingredients in the specified proportions and, if necessary, adjusting the pH to the specified range.

**Patentansprüche**

1. Verdickte wässerige Natriumfluorid-Zusammensetzung, die sich zur Anwendung in der topischen prophylaktischen Behandlung von Zähnen eignet, welche Zusammensetzung 75,2 bis 96,1 Gew.-% Wasser, 1 bis 5 Gew.-% Natriumfluorid umfaßt, und worin das Verdickungsmittel aus Xanthangummi und einem wasserlöslichen Salz eines Acrylsäurepolymers besteht, welche Zusammensetzung einen pH-Wert von 6 bis 8 aufweist.

2. Zusammensetzung nach Anspruch 1 mit einem zusätzlichen Gehalt an einem Puffermittel.

3. Zusammensetzung nach Anspruch 2, worin das Puffermittel Dinatriumphosphat, Monoatriumphosphat oder ein Gemisch hievon ist.

4. Zusammensetzung nach einem der Ansprüche 1 bis 3, mit einem weiteren Gehalt an Geschmacksmitteln, Konservierungsmitteln oder Färbemitteln.

5. Zusammensetzung nach einem der Ansprüche 1 bis 4, welche eine Viskosität von 4 bis 20 Pa.S (40 bis 200 Poise), bestimmt auf einem Brookfield-Viskometer bei 60 Umdrehungen je Minute und 25°C, und eine Viskosität von 50 bis 400 Pa.S (500 bis 4000 Poise), bestimmt auf einen Brookfield-Viskometer bei 1,5 Umdrehungen je Minute und 25°C, aufweist.

6. Zusammensetzung nach einem der Ansprüche 1 bis 5, worin das Verdickungsmittel Xanthangummi in Verbindung mit dem Natriumsalz der Polyacrylsäure ist.

7. Zusammensetzung nach einem der Ansprüche 1 bis 6 zur Anwendung in der Bekämpfung von Dentalkaries.

8. Verfahren zur Herstellung einer Zusammensetzung nach einem der Ansprüche 1 bis 7, umfassend ein Vermischen der genannten Bestandteile in den angegebenen Verhältnissen und erforderlichenfalls ein Einstellen des pH-Wertes auf den angegebenen Bereich.

**Revendications**

1. Composition aqueuse épaissie de fluorure de sodium qui convient pour être utilisée dans le traitement topique prophylactique des dents, ladite composition comprenant 75,2 à 96,1% poids/poids d'eau, 1 à 5% poids/poids de fluorure de sodium et un épaississant comprenant de la gomme de xanthane et un sel soluble dans l'eau d'un polymère d'acide acrylique, ladite composition ayant un pH de 6 à 8.

2. Composition selon la revendication 1, qui comprend en outre un tampon.

3. Composition selon la revendication 2, dans laquelle le tampon est le phosphate disodique, le phosphate monosodique ou un de leurs mélanges.

4. Composition selon l'une quelconque des revendications 1 à 3, qui comprend en outre des substances aromatiques, des conservateurs ou des colorants.

5. Composition selon l'une quelconque des revendications 1 à 4, qui a une viscosité allant de 4 à 20 Pa.s (40 à 200 poises), évaluée à l'aide d'un viscosimètre de Brookfield à 60 tr/min et 25°C, et une viscosité de 50 à 400 Pa.s (500 à 4000 poises) évaluée à l'aide d'un viscosimètre de Brookfield à 1,5 tr/min et 25°C.

6. Composition selon l'une quelconque des revendications 1 à 5, dans laquelle l'épaississant est la gomme de xanthane plus le sel de sodium de l'acide polyacrylique.

7. Composition selon l'une quelconque des revendications 1 à 6 à utiliser dans la lutte contre la carie dentaire.

8. Procédé de préparation d'une composition selon l'une quelconque des revendications 1 à 7, qui comprend le mélange desdits ingrédients dans les proportions déterminées, et si nécessaire, le réglage du pH dans l'intervalle spécifié.